# FASCICULE DE BREVET EUROPEEN

(11) **EP 1 292 233 B1**
(45) Date de publication et mention de la délivrance du brevet: **21.03.2007**
(21) Numéro de dépôt: 01932028.2
(22) Date de dépôt: 29.05.2001
(51) Int. Cl.: A61B 17/04, A61F 2/08

(54) **ENSEMBLE DE FIXATION D'UN TISSU MOU SUR UN OS**
VERFAHREN ZUR BEFESTIGUNG EINES WEICHEN GEWEBES AUF EINEM KNOCHEN
METHOD FOR FIXING A SOFT TISSUE ON A BONE

(30) Priorité: 31.05.2000 CH 10962000
(43) Date de publication de la demande: 19.03.2003
(73) Titulaire: MD Supply S.à.r.l., 1400 YVERDON-LES-BAINS (CH)
(72) Inventeur: FRIDEN,Per-Anders, CH-1003 Lausanne (CH)
(74) Mandataire: Nithardt, Roland
(86) Numéro de dépôt international: PCT/IB2001/000959
(87) Numéro de publication internationale: WO 2001/091645

(56) Documents cités:
- EP-A- 0 599 772
- EP-A- 0 770 354
- EP-A- 0 916 311
- EP-A- 1 070 487
- US-A- 4 438 769
- US-A- 5 354 292
- US-A- 6 149 669
- US-B1- 6 187 009
- US-B1- 6 190 401

## Description

### Domaine technique

La présente invention concerne un ensemble de fixation d'un tissu mou, notamment d'un ligament ou d'un tendon, sur un os au moyen d'au moins deux clous, chaque clou comportant une portion supérieure cylindrique et une portion inférieure formée par une superposition de cônes inversés ayant une directrice dont le diamètre est supérieur au diamètre de la portion cylindrique, cet ensemble comprenant un moyen de liaison de deux clous, des moyens de maintien en position du tissu mou sur l'os après la mise en place des clous, chaque clou ayant une forme agencée pour empêcher son déplacement vertical dans l'os après sa mise en place, ainsi qu'un organe de mise en place, simultanément dans le tissu mou et l'os superposés, des deux clous reliés par le moyen de liaison, avec un écartement déterminé, le moyen de liaison étant un fil de suture, ledit organe de mise en place des clous comportant un manche dans lequel est emboîtée une tige cylindrique.

### Technique antérieure

Les lésions ligamentaires sont de plus en plus fréquentes lors des nombreux accidents se produisant notamment dans le domaine sportif. Il convient alors de procéder à une reconstruction ligamentaire qui est durable dans le temps.

Dans la plupart des interventions chirurgicales orthopédiques pratiquées actuellement, la fixation du ligament ou du tendon déchiré au bloc osseux se fait à l'aide d'agrafes telles que couramment utilisées en chirurgie. Cependant, lorsqu'on utilise de telles agrafes, il y a toujours une partie métallique qui dépasse de la surface du tissu mou et qui risque de provoquer une nécrose des tissus.

Ce type de fixation peut aussi se faire à l'aide de vis appropriées. Un des inconvénients majeurs de ces vis est qu'il faut percer l'os pour les implanter.

Un autre inconvénient est que l'endroit exact du positionnement est difficile à déterminer

Une première forme de réalisation d'un des éléments de fixation connus est décrite dans la publication européenne EP 0 770 354 qui a pour objet un appareil qui se comporte comme une agrafeuse, mais se présente sous la forme d'un pistolet pourvu d'un logement longitudinal formant un magasin dans lequel sont stockés des éléments de fixation emboîtés l'un dans l'autre. Ce dispositif est exclusivement destiné à la réparation de tissus déchirés dans le ménisque du genou. En effet, les agrafes sont réalisées à base d'acide lactique, sont résorbables et ne peuvent pas être implantées dans un os. De plus, par construction, l'écartement entre deux éléments de fixation est nécessairement fixe, ce qui rend l'invention inutilisable pour une grande variété d'applications. Comme mentionné précédemment, l'existence d'une liaison semi-rigide entre les deux éléments de fixation peut générer une nécrose des tissus. Finalement, il n'est possible de lier les éléments de fixation que deux par deux et il n'est pas possible de lier trois ou quatre éléments entre eux, ce qui peut s'avérer nécessaire lorsque la surface d'attache est grande.

L'instrument chirurgical objet de la publication EP 0 589 306 se présente également sous la forme d'une agrafeuse destinée à fixer un tissu mou sur un ménisque. Les attaches utilisées dans cette agrafeuse comportent deux branches reliées par un élément flexible. L'écartement des branches défini par la longueur de cet élément flexible est fixe. En outre, les branches de l'attache sont enfoncées l'une après l'autre dans le tissu.

Une autre forme de réalisation d'un élément de fixation est décrite dans le brevet US 5 012 421. Ce document concerne un clou permettant l'ancrage de l'extrémité d'un fil de suture dans un os. Ce clou comporte une portion supérieure cylindrique dans laquelle est emprisonnée l'extrémité du fil et une portion inférieure formée par une superposition de cônes inversés. Ce clou est mis en place à l'aide d'un outil comportant, à son extrémité en contact avec l'os, une cavité cylindrique borgne agencée pour recevoir la partie cylindrique du clou. Ce type d'élément a notamment pour inconvénient le fait que, de par sa taille, il détruit les tissus lors de son enfoncement. Il ne peut donc pas être introduit à travers un tissu, mais doit être placé à côté de ce tissu. La mise en place est donc difficile. Ce clou comporte en outre des gorges en spirale qui le font tourner sur lui-même lors de sa mise en place dans l'os. Ceci empêche d'utiliser "l'élasticité" de l'os qui entraîne que, lorsque le clou est chassé dans l'os, celui-ci s'étend pour laisser le clou s'enfoncer, puis reprend sa position initiale pour maintenir très fermement le clou. Le fait que le fil de suture soit lié au clou implique qu'il n'est pas possible d'utiliser un fil avec lequel les utilisateurs ont l'habitude de travailler. De plus, lorsque le fil est utilisé pour fixer un tissu mou, il n'existe qu'un seul pont entre les deux clous. Or, dans les opérations ayant échoué, une grande proportion des échecs sont dus à la rupture du tissu par le fil qui doit d'une part être suffisamment tendu pour maintenir le tissu et d'autre part suffisamment détendu pour ne pas le cisailler.

La publication EP 0 835 640 décrit un dispositif d'ancrage, dans une substance osseuse, d'un fil de suture équipé d'une aiguille à chacune de ses extrémités. Pour éviter un perçage préalable de l'os, l'élément support recevant le fil et destiné à être ancré dans la substance osseuse comporte une zone d'extrémité pointue faisant office de pointeau, une zone tronconique filetée pour assurer la fonction d'autotaraudage et une zone cylindrique pour assurer l'ancrage dans l'os. Le dispositif de mise en place de cet élément se présente sous la forme d'un tournevis, l'élément support étant introduit dans l'os par autotaraudage, formé d'un manche et d'une tige dont l'extrémité libre est agencée pour coopérer avec la partie supérieure de l'élément support. Le manche du dispositif comporte un évidement destiné au logement de l'ensemble fil et aiguilles et la tige est pourvue d'une fente longitudinale destinée à recevoir les deux brins du fil lors de la mise en place de l'élément support. Ce dispositif, comme le précédent, ne permet pas de faire deux ancrages dans une même étape, ce qui ne garantit pas un parallélisme entre deux éléments enfoncés l'un après l'autre. Le manque de parallélisme implique un arrachement facilité.

Les dispositifs de l'art antérieur permettent soit d'introduire deux éléments de fixation en même temps, avec un écartement qui ne peut pas être modifié, soit d'introduire un seul élément de fixation à la fois, qui tourne pendant son introduction. De plus, ils réalisent une compression inadaptée à la surface du tissu, ce qui risque d'entraîner soit une nécrose, soit une rupture du tissu.

### Exposé de l'invention

La présente invention se propose de pallier les inconvénients des dispositifs de l'art antérieur en offrant un ensemble permettant une fixation précise et rapide d'un tissu mou sur un os, sans élément métallique apparent et sans perçage de l'os. Cet ensemble offre une large surface de contact et de cicatrisation entre le tissu et l'os sans risque de nécrose des tissus et permet une fixation sûre sans risque d'arrachement des clous dû à une mise en place approximative de ces clous. De plus, le risque de cisaillement des tissus par le fil de suture est pratiquement inexistant.

A cet effet, cet ensemble, tel que défini en préambule, est caractérisé en ce que l'extrémité de la tige cylindrique de l'organe de mise en place des clous comporte une forme de fourche ayant deux branches dont la distance entre les axes longitudinaux est égale audit écartement déterminé des axes longitudinaux des clous, chaque branche comportant un alésage cylindrique borgne, agencé pour coopérer avec la portion cylindrique d'un clou, et deux rainures latérales agencées pour recevoir le fil de suture, et en ce que ledit manche comporte deux éléments d'arrêt diamétralement opposés agencés pour retenir le fil de suture lorsque les clous, reliés par ledit fil de suture, sont solidaires de l'extrémité de la tige cylindrique de l'organe de mise en place des clous afin d'être pointés sur le tissu mou disposé au-dessus de l'os.

De préférence, la portion cylindrique d'un clou comporte un alésage cylindrique transversal agencé pour coopérer avec le moyen de liaison des clous.

Dans un mode de réalisation préféré, l'extrémité de la tige cylindrique dudit organe de mise en place des clous comporte des moyens de contrôle de la profondeur d'enfoncement desdits clous dans l'os.

L'ensemble selon l'invention peut en outre comporter un élément de positionnement des clous avant leur saisie par l'organe de mise en place, ledit élément de positionnement étant agencé pour obtenir l'écartement déterminé requis entre les axes longitudinaux des deux clous.

De façon avantageuse, cet élément de positionnement comporte deux logements agencés pour recevoir la portion inférieure des clous, les axes longitudinaux de ces logements étant écartés d'une distance égale audit écartement déterminé requis des axes longitudinaux desdits clous, et il comporte également des moyens pour maintenir les clous dans lesdits logements, dans une position définie.

### Description sommaire des dessins

La présente invention sera mieux comprise en référence à la description d'une forme de réalisation préférée et non limitative, et des dessins annexés dans lesquels :
- la figure 1 est une vue en élévation d'un des clous utilisés dans l'ensemble selon l'invention;
- la figure 2 représente une vue en perspective d'un l'élément de positionnement des clous;
- la figure 3 illustre l'organe de mise en place des deux clous ainsi que le moyen de liaison de ces clous;
- la figure 4 est une vue agrandie selon la flèche A de l'organe de mise en place de la figure 3;
- les figures 5A et 5B sont des représentations agrandies respectivement de face et latérale de l'extrémité de préhension des clous de l'organe de mise en place de la figure 3, et
- la figure 6 illustre respectivement les différentes étapes du procédé selon l'invention.

### Meilleure manière de réaliser l'invention

En référence aux figures, l'ensemble selon l'invention comprend deux clous 10 destinés à solidariser un tissu mou, tel que l'extrémité d'un tendon ou d'un ligament, sur un bloc osseux lors d'une intervention chirurgicale réparatrice. Afin de faciliter la mise en place de ces deux clous en les enfonçant simultanément dans l'os avec un écartement déterminé, comme lors de l'utilisation d'agrafes, l'ensemble, tel qu'illustré par les figures, comprend en outre un élément de positionnement 20 des clous 10 avant leur saisie par un organe de mise en place 30 desdits clous dans le tissu mou et l'os superposés. Ces deux clous sont liés par un moyen de liaison 40 formé d'un fil de suture destiné à former, après leur mise en place, des moyens de maintien en position 50 du tissu mou sur l'os.

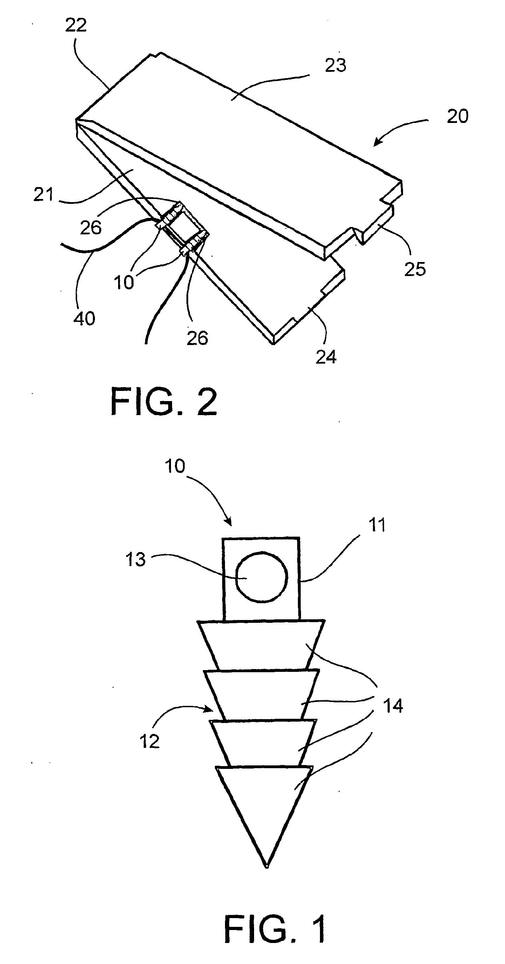

Les clous 10, tels que celui illustré par la figure 1, et dont la forme «en sapin inversé» a été spécialement étudiée pour obtenir une fixation solide, comporte une première portion supérieure cylindrique 11 et une seconde portion inférieure 12 formée d'une superposition coaxiale «en escalier» de cônes inversés 14. La portion cylindrique 11 est pourvue d'un alésage cylindrique 13 chanfreiné, ménagé transversalement et dont la fonction sera précisée ci-après. Les cônes 14 de la portion inférieure 12 sont formés par une génératrice faisant un angle sensiblement égal à 60° par rapport à l'axe vertical médian du clou et dont le diamètre de la directrice est supérieur au diamètre de la portion cylindrique 11. Afin de faciliter la perforation du tissu mou, la génératrice du cône 14 formant l'extrémité du clou fait un angle sensiblement égal à 50° par rapport à l'axe vertical.

L'élément de positionnement 20 des deux clous 10, tel qu'illustré par la figure 2, et dont l'utilisation n'est pas obligatoire dans le procédé de l'invention, est agencé pour obtenir l'écartement déterminé requis entre les axes longitudinaux de ces deux clous 10. Il se présente sous la forme d'une pince comportant une partie inférieure 21 reliée, par une charnière 22, à une partie supérieure 23. Les deux parties 21 et 23 sont de forme parallélépipédique.. rectangle et comportent respectivement une face inférieure et supérieure planes destinées à être appuyées l'une contre l'autre. L'élément de positionnement 20 comporte en outre des moyens de fermeture pour maintenir ces deux faces en contact. A cet effet, la partie inférieure 21 est pourvue, dans sa face latérale opposée à la charnière 22, d'un rebord saillant 24 destiné à coopérer avec un rebord 25 correspondant, en forme de crochet, ménagé sur la partie supérieure 23. La partie inférieure 21 est en outre pourvue de deux logements 26 destinés à recevoir les deux clous 10. Ces logements sont disposés l'un à côté de l'autre et leurs axes longitudinaux sont écartés d'une distance égale à l'écartement déterminé des axes longitudinaux des deux clous lors de leur mise en place dans l'os. La profondeur et la largeur de ces logements 26 sont respectivement égales au diamètre de la directrice des cônes 14 et leur longueur est égale à la longueur de la portion inférieure 12 des clous 10. Ainsi, lorsque le dispositif est refermé après la mise en place des clous dans les logements 26, les alésages 13 étant placés dans le prolongement l'un de l'autre, seules les parties cylindriques 11 dépassent de l'élément 20. Les deux clous 10 sont alors liés par le moyen de liaison 40 que l'on fait passer respectivement dans chaque alésage 13.

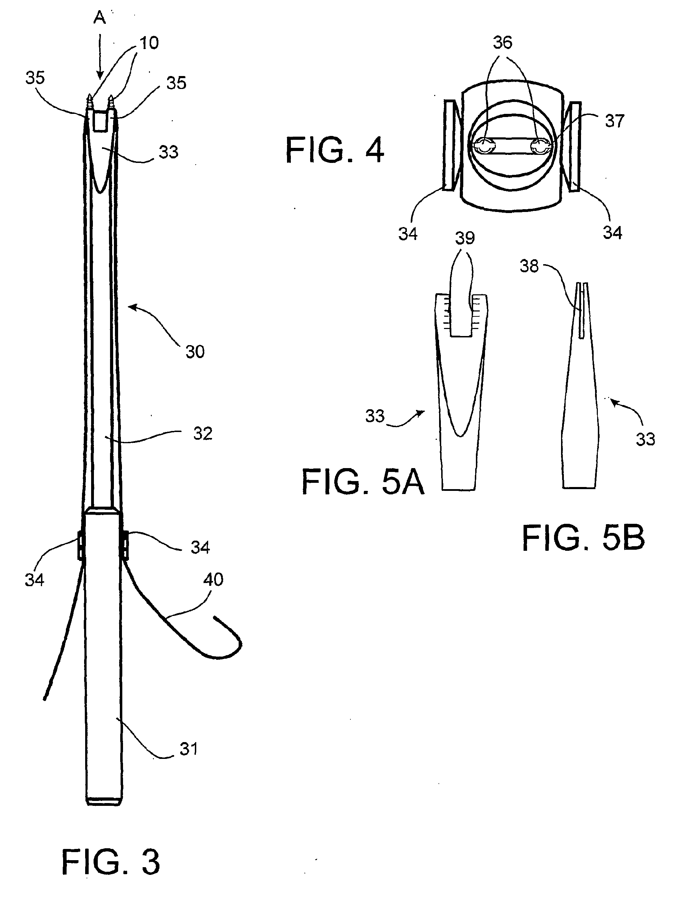

Afin de permettre la présentation des clous 10 ainsi positionnés et équipés du fil de suture 40 au-dessus du tissu mou placé sur l'os, on utilise l'organe de mise en place 30 tel qu'illustré par les figures 3 à 5.

Cet organe de mise en place 30, tel que représenté par la figure 3, comprend un manche 31 qui peut avoir une section sensiblement carrée (comme illustré) ou cylindrique, de préférence réalisé en acier inoxydable, et dans lequel est emboîtée une tige cylindrique 32, de préférence en acier inoxydable, comportant une extrémité 33, de forme ovoïde, qui sera décrite en référence aux figures 4 et 5. Ce manche 31 est par ailleurs pourvu, sur deux de ses faces, de deux éléments d'arrêt coniques 34 diamétralement opposés, dont la fonction sera précisée ci-après.

L'extrémité 33 de la tige 32, représentée plus en détail par les figures 4, 5A et 5B, se présente sous la forme d'une fourche en forme de U comportant deux branches 35 dont la distance entre les axes longitudinaux est égale à l'écartement déterminé des axes longitudinaux de ces clous. Par ailleurs, chaque branche 35 est pourvue d'un alésage cylindrique borgne 36 dont le diamètre est sensiblement supérieur à celui de la partie cylindrique 11 des clous et dont la profondeur est au moins égale à la hauteur de cette partie 11. Une fente transversale 37 médiane est ménagée à travers ces alésages 36. Cette fente 37 se prolonge de chaque côté de chaque branche 35 par deux rainures verticales 38, et de préférence incurvées, illustrées par la figure 5B. Les bords intérieurs des branches 35 formant les côtés du U sont pourvus d'une graduation millimétrique formant des moyens de contrôle 39 de la profondeur d'enfoncement des clous dans l'os, comme cela est illustré par la figure 5A.

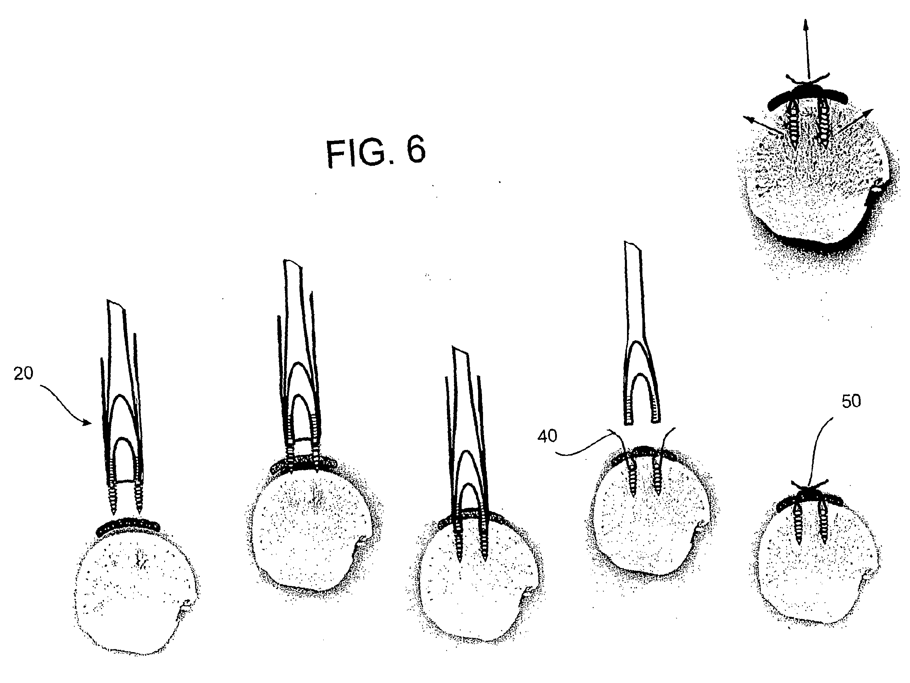

Les différentes étapes de l'utilisation du ensemble selon l'invention, telles qu'illustrées par la figure 6, se déroulent de la façon suivante :
- lorsque l'on utilise l'élément de positionnement 20, cet élément étant ouvert, on place la portion 12 des clous 10 dans les logements 26 prévus à cet effet, la portion cylindrique 11 dépassant du bord de la partie inférieure 21. On referme l'élément 20, le rebord en forme de crochet 25 étant accroché au rebord saillant 24, afin d'immobiliser les clous dans leur logement. Les alésages 13 de la portion cylindrique 11 des clous ayant été disposés dans le prolongement l'un de l'autre lors de cette mise en place, on les relie en enfilant le fil de suture 40 dans les deux alésages 13.
- les clous 10 et le fil 40 étant en place, on prend l'organe de mise en place 30 par le manche 31 et l'on applique l'extrémité 33 sur les clous, les parties cylindriques 11 entrant dans les trous borgnes 36 des deux branches 35 et le fil de suture 40 se logeant alors dans la fente 37. Pour que les clous ne se désolidarisent pas de l'extrémité 33 lors de l'ouverture de l'élément de positionnement 20, on enroule les extrémités libres du fil de suture 40 autour des éléments d'arrêt 34 disposés sur le manche en faisant passer le fil dans les rainures latérales extérieures 38.
- le tissu mou à fixer étant positionné au-dessus du bloc osseux, on pointe ce tissu à l'endroit où il doit être ressoudé à l'os et l'on positionne l'extrémité des clous sur ce point en tenant l'ensemble formé par l'organe de mise en place 30 et les clous 10 selon la normale à la surface de l'os. On peut alors enfoncer les clous en tapant à l'aide d'un marteau approprié sur l'extrémité libre du manche 31. On contrôle la profondeur de l'enfoncement à l'aide de la graduation des moyens de contrôle 39 de l'extrémité 33. La forme en U de cette extrémité 33 permet au tissu mou de ne pas être endommagé entre les deux clous 10 puisqu'il vient se loger entre les deux branches 35, le fil 40 qui les relie maintenant le tissu en position lors de cette opération. En outre, les rainures 38 protègent le fil de suture 40 lors de cet enfoncement.
- lorsque les clous 10 sont enfoncés, on les désolidarise de l'organe de mise en place 30 en libérant le fil de suture 40 dont on noue les deux extrémités pour former un noeud 51 qui constitue les moyens 50 de maintien en position du tissu mou sur l'os. Cette double liaison par le fil permet d'éliminer le risque de cisaillement des tissus.

De par sa conception, ce mode de fixation est fiable. En effet, d'une part la forme particulière «en sapin» des clous permet une compression de l'os et, de ce fait, une réaction biologique d'auto-blocage du clou. D'autre part, les clous étant reliés par un fil, toute tension exercée sur ce fil tend à modifier le parallélisme des clous dont les pointes s'écartent alors vers l'extérieur, ce qui renforce la solidité de la fixation.

En outre, il faut noter que cet ensemble assure le parallélisme des clous lors de leur enfoncement et garantit qu'aucune partie métallique ne dépasse la surface de l'os au terme de l'opération. Lorsque les deux clous ne sont pas dans un même plan le risque d'arrachement est augmenté. De par sa conception, ce type de fixation peut aussi bien être utilisé en chirurgie ouverte qu'en endoscopie.

L'utilisation de tels clous permet également d'utiliser n'importe quel type de fil de suture auquel l'utilisateur est habitué. Il est également possible, lors d'opérations sur une surface importante, d'utiliser quatre clous ou plus et d'effectuer des liaisons entre tous ces clous. Ceci permet une fixation optimale des tissus sur l'os.

Lorsque le risque de cisaillement des tissus est important, il est possible d'effectuer plusieurs passages de fils dans les clous, ce qui augmente la surface de maintien des tissus et diminue le risque de rupture.

Ainsi, grâce à l'utilisation des clous et de l'ensemble selon l'invention, l'intervention est simplifiée et est plus rapide puisqu'il est possible de tenir le tissu mou avec l'extrémité des clous, de le fixer en enfonçant les clous et de nouer les fils très rapidement, tout en s'adaptant à une grande variété d'opérations différentes.

## Revendications

1. Ensemble de fixation d'un tissu mou, notamment d'un ligament ou d'un tendon, sur un os au moyen d'au moins deux clous, chaque clou (10) comportant une portion supérieure cylindrique (11) et une portion inférieure (12) formée par une superposition de cônes inversés (14) ayant une directrice dont le diamètre est supérieur au diamètre de la portion cylindrique (11), cet ensemble comprenant un moyen de liaison (40) de deux clous, des moyens de maintien en position (50) du tissu mou sur l'os après la mise en place des clous, chaque clou (10) ayant une forme agencée pour empêcher son déplacement vertical dans l'os après sa mise en place, ainsi qu'un organe de mise en place (30), simultanément dans le tissu mou et l'os superposés, des deux clous (10) reliés par le moyen de liaison, avec un écartement déterminé, le moyen de liaison étant un fil de suture (40), ledit organe de mise en place (30) des clous (10) comportant un manche (31) dans lequel est emboîtée une tige cylindrique (32), l'extrémité (33) de la tige cylindrique (32) de l'organe de mise en place (30) des clous (10) comportant une forme de fourche ayant deux branches (35) dont la distance entre les axes longitudinaux est égale audit écartement déterminé des axes longitudinaux des clous (10), **caractérisé en ce que** chaque branche (35) comporte un alésage cylindrique borgne (36), agencé pour coopérer avec la portion cylindrique (11) d'un clou (10), et deux rainures latérales (38) agencées pour recevoir le fil de suture (40), et **en ce que** ledit manche (31) comporte deux éléments d'arrêt (34) diamétralement opposés agencés pour retenir le fil de suture (40) lorsque les clous (10), reliés par ledit fil de suture, sont solidaires de l'extrémité (33) de la tige cylindrique (32) de l'organe de mise en place (30) desdits clous (10) afin d'être pointés sur le tissu mou disposé au-dessus de l'os.

2. Ensemble selon la revendication 1, **caractérisé en ce que** la portion cylindrique (11) d'un clou (10) comporte un alésage cylindrique transversal (13) agencé pour coopérer avec le moyen de liaison (40).

3. Ensemble selon la revendication 1, **caractérisé en ce que** l'extrémité (33) de la tige cylindrique (32) dudit organe de mise en place (30) comporte des moyens de contrôle (39) de la profondeur d'enfoncement des clous (10) dans l'os.

4. Ensemble selon la revendication 1, **caractérisé en ce qu'**il comporte en outre un élément de positionnement (20) des clous avant leur saisie par l'organe de mise en place (30), ledit élément de positionnement (20) étant agencé pour obtenir l'écartement déterminé requis entre les axes longitudinaux des deux clous (10).

5. Ensemble selon la revendication 4, **caractérisé en ce que** l'élément de positionnement (20) comporte deux logements (26) agencés pour recevoir la portion inférieure (12) des clous (10), les axes longitudinaux de ces logements étant écartés d'une distance égale audit écartement déterminé requis des axes longitudinaux des desdits clous (10), et **en ce qu'**il comporte des moyens pour maintenir les clous (10), dans lesdits logements (26), dans une position définie.

## Claims

1. A device for fixing a connective tissue, notably a ligament or a tendon, to a bone by at least two pins, each pin (10) comprising a superior section (11) and an inferior section (12) provided with superimposed inverted cones (14) whose directrix diameter is superior to the diameter of the cylindrical portion (11), this device comprising a connecting means (40) for connecting two pins, a maintaining mechanism (50) for maintaining connective tissue on the bone after the positioning of the pins, each pin (10) having a shape designed to prevent its vertical moving into the bone after its positioning, and a maintaining in position mechanism (30) for positioning, simultaneously into the connective tissue and the bone superimposed, the two pins (10) connected by the connecting means, with a defined space, the connecting means comprising a suture thread (40), said positioning and maintaining in position mechanism (30) of the pins (10) comprising a shaft (31) in which a cylindrical stem (32) is inserted, the end part (33) of the cylindrical stem (32) of the positioning and maintaining in position mechanism (30) being designed like a fork with two branches (35), the distance between the longitudinal axes of said branches being equal to said defined space of the pins (10), **characterized in that** each branch (35) comprises a cylindrical transverse bore (36) adapted to cooperate with the cylindrical superior section (11) of a pin (10) and with two lateral grooves (38) designed to receive the suture thread (40), and **in that** said shaft (31) comprises two stopping elements (34) that are diametrically opposite, designed to hold said suture thread (40) when the pins (10), connected by said suture thread, are attached to the end part (33) of the cylindrical stem (32) of the positioning and maintaining in position mechanism (30) in order to be directed to the connective tissue situated above the bone.

2. The device according to claim 1, **characterized in that** the superior section (11) of a pin (10) comprises a transverse cylindrical bore (13) designed to cooperate with the connecting means (40).

3. The device according to claim 1, **characterized in that** the end part (33) of the cylindrical stem (32) of said positioning and maintaining in position mechanism (30) comprises a means (39) for controlling the depth of insertion of the pins (10) in the bone.

4. The device according to claim 1, **characterized in that** it further comprises a prepositioning element (20) for the pins before they are taken again by the positioning and maintaining in position mechanism (30), said prepositioning element (20) being designed to obtain the defined space between the longitudinal axes of the two pins (10).

5. The device according to claim 4, **characterized in that** the prepositioning element (20) comprises two housings (26) designed to receive the inferior section (12) of the pins (10), the longitudinal axes of the these housings being spaced at a distance equal to said defined space of longitudinal axes of said pins (10), and **in that** said device comprises a means for maintaining the pins (10) in said housings (26), in a defined position.

## Patentansprüche

1. Befestigungseinheit für ein weiches Gewebe, insbesondere eines Bandes oder einer Sehne auf einem Knochen mittels mindestens zweier Nägel, wobei jeder Nagel (10) einen oberen zylindrischen Abschnitt (11) und einen unteren Abschnitt (12) aufweist, der aus einer Übereinanderlagerung umgekehrter Kegel (14) ausgebildet ist, die eine Leitlinie aufweisen, deren Durchmesser über dem Durchmesser des zylindrischen Abschnittes (11) liegt, wobei die Einheit eine Verbindungseinrichtung (40) für zwei Nägel und Einrichtungen (50) zum Halten des weichen Gewebes in seiner Position auf dem Knochen nach der Platzierung der Nägel aufweist, wobei jeder Nagel (10) eine Form aufweist, die derart ist, dass sie dessen Vertikalverlagerung in dem Knochen nach seiner Platzierung verhindert, sowie ein Element (30) zur gleichzeitigen Platzierung der zwei Nägel (10), die durch die Verbindungseinrichtung verbunden sind, in dem übereinandergelagerten weichen Gewebe und dem Knochen mit einem bestimmten Abstand, wobei die Verbindungseinrichtung ein Nähfaden (40) ist, wobei das Element (30) zur Platzierung der Nägel (10) einen Handgriff (31) aufweist, in dem eine zylindrische Stange (32) eingepasst ist, wobei das äußerste Ende (33) der zylindrischen Stange (32) des Elementes (30) zur Platzierung der Nägel (10) eine Gabelform mit zwei Schenkeln (35) aufweist, deren Abstand zwischen den Längsachsen gleich dem bestimmten Abstand der Längsachsen der Nägel (10) ist,
**dadurch gekennzeichnet , dass**
jeder Schenkel (35) eine zylindrische Blindbohrung (36), die angeordnet ist, um mit dem zylindrischen Abschnitt (11) eines Nagels (10) zusammenzuwirken, und zwei seitliche Nuten (38) aufweist, die angeordnet sind, um den Nähfaden (40) aufzunehmen, und dass der Handgriff (31) zwei einander diametral gegenüberliegende Halteelemente (34) aufweist, die angeordnet sind, um den Nähfaden (40) zurückzuhalten, wenn die durch den Nähfaden verbundenen Nägel (10) fest mit dem äußersten Ende (33) der zylindrischen Stange (32) des Elementes (30) zur Platzierung der Nägel (10) verbunden sind, um durch das über dem Knochen angeordnete weiche Gewebe hindurchgestochen zu werden.

2. Einheit nach Anspruch 1,
**dadurch gekennzeichnet , dass**
der zylindrische Abschnitt (11) eines Nagels (10) eine transversale zylindrische Bohrung (13) aufweist, die zum Zusammenwirken mit der Verbindungseinrichtung (40) angeordnet ist.

3. Einheit nach Anspruch 1,
**dadurch gekennzeichnet , dass**
das äußerste Ende (33) der zylindrischen Stange (32) des Elementes (30) zur Platzierung Einrichtungen (39) zur Steuerung der Einschlagtiefe der Nägel (10) in dem Knochen aufweist.

4. Einheit nach Anspruch 1,
**dadurch gekennzeichnet , dass** sie außerdem ein Element (20) zur Positionierung der Nägel vor deren Ergreifen durch das Element (30) zur Platzierung aufweist, wobei das besagte Element (20) zur Positionierung angeordnet ist, um den Abstand zu erreichen, der zwischen den Längsachsen der zwei Nägel (10) bestimmt ist.

5. Einheit nach Anspruch 4,
**dadurch gekennzeichnet , dass**
das Element (20) zur Positionierung zwei Aufnahmen (26) aufweist, die zur Aufnahme des unteren Abschnittes (12) der Nägel (10) vorgesehen sind, wobei die Längsachsen dieser Aufnahmen mit einem Abstand beabstandet sind, der gleich dem bestimmten erforderlichen Abstand der Längsachsen der besagten Nägel (10) ist, und dass sie Einrichtungen zum Halten der Nägel (10) in den besagten Aufnahmen (26) in einer festgelegten Position aufweist.
